# EUROPEAN PATENT APPLICATION

(11) **EP 2 248 516 A1**
(43) Date of publication of application: **10.11.2010**
(21) Application number: 10162059.9
(22) Date of filing: 05.05.2010
(51) Int. Cl.: A61K 9/00, A61K 9/20, A61K 31/44

(54) **Orally disintegrating compositions of emoxypine**

(30) Priority: 06.05.2009 TR 200903548
(71) Applicant: Sanovel Ilac Sanayi ve Ticaret A.S., 34398 Istanbul (TR)
(72) Inventor: Toksöz, Ahmet, 34398, Istanbul (TR); Cifter, Ümit, 34398, Istanbul (TR); Türkyilmaz, Ali, 34398, Istanbul (TR)
(74) Representative: Sevinç, Erkan

(57) **Abstract**

The present invention is related to an orally disintegrating composition comprising emoxypine or a pharmaceutically acceptable salt thereof and one or more pharmaceutically acceptable excipient.

## Description

### Technical Aspect

The present invention is related to an orally disintegrating composition comprising emoxypine or a pharmaceutically acceptable salt thereof and one or more pharmaceutically acceptable excipient.

### Background of the Invention

Emoxypine is a heteroaromatic antioxidant which has a membranoprotective, nootropic and sedative action. Its chemical name is 2-ethyl-6-methyl-3-hydroxypyridine and its chemical structure is shown in the Formula I.

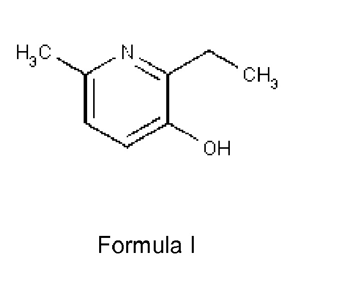

Its aromatic activity range is very wide such as increasing the stress resistance of organism. It acts as the anxiolytic which doesn't cause sleepiness or doesn't have myorelaxant effect; its nootropic properties prevent and reduce learning and memory defects in patients of old age under pathogenic factors. One of the activities of emoxypine is its anticonvulsant activity which shows antioxidant and antihypoxic properties. It increases the attention concentration and capacity for work and decreases the toxic affect of alcohol. It inhibits peroxide oxidation of lipids, increases the superoxidoxidase activity and elevates the ratio of lipid-protein. Also, provides a higher dopamine concentration in brain.

Emoxypine is marketed under the brand name MEXIDOL^{®}and it is administered orally in a therapeutic dose from 125 mg to 250 mg, three times a day and it has injectable formulations containing 50mg/ml in 2 ml and 5ml ampules in IV/IM forms.

Injectable and drop solutions of emoxypine HCl were disclosed in US patent no 4,486,440 in 17.08.1983. They are used as retinoprotector for treating intraocular hemorrhage, myopic chorioretinal dystrophies, congenital retinal dystrophies, retinal burns and prevention of injury in lasercoagulation.

A conventional tablet formulation of emoxypine succinate (Mexidol^{®}) is described in PCT application WO 96/02238 A1, Aktsionernoe Obschestvo Zakrytogo Tipa 'Olvik', 15.07.1994. The proposed neurotropic and sedative formulation contains emoxypine succinate and auxiliary agents in core and a coating layer. The auxiliary agents in the core comprise kaolin, potato starch, calcium stearate, stearic acid, talc and methyl cellulose. The coating layer contains titanium dioxide and serge 80.

In prior art, there are also several patents which disclose orally disintegrating compositions but none of them includes emoxypine or a pharmaceutically acceptable salt thereof.

Orally disintegrating dosage forms are becoming an increasingly important issue in the area of better patient compliance comparative to the conventional solid dosage forms for oral administration such as capsules and tablets, which are the most commonly used. In particular pediatric and geriatric patients, and patients with mental problems and non- compliant patients often experience difficulties in swallowing solid dosage forms. Besides, conventional solid dosage forms are not suitable for bedridden or busy and travelling patients, in case the patient may not have easy access to water. Thus, orally disintegrating compositions represent an alternative for such patients and provide for a better patient compliance with recommended pharmaceutical therapies. In addition, medicaments which may be used for sedatives, hypnotics, and antipsychotic are amendable such dosage forms.

Additionally oral administration of the drugs is difficult in patients having concomitant vomiting, nausea or diarrhoea. The orally disintegrating dosage form is one of the advantageous methods to deliver the drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of first-pass liver metabolism.

It is difficult to develop orally disintegrating compositions because of several different reasons. A satisfied orally disintegrating dosage form needs to meet a number of requirements. Firstly, it has to disintegrate in the mouth spontaneously. The time in which a dosage form must dissolve or disintegrate in the oral cavity is necessarily much shorter than in the stomach. Moreover, a premature release in the mouth could also lead to problems due to the often unpleasant taste of the active ingredient. Besides, these compositions should be very porous and should not be very hard. These porous compositions tend to be very sensitive to humidity. As a consequence, they may have some stability problems.

To fulfill all these requirements the formulation for a specific drug needs to be adapted in particular by a careful selection of the excipients used. However, the excipients selected may lead to formulations which are not bioavailable to the corresponding conventional dosage forms. Thus, they have to be chosen very carefully.

Additionally, precautions have to be taken at the preparation, packaging, handling and storing of the finished dosage forms of orally disintegrating compositions since they tend to be both hygroscopic and friable.

Thus, a need rises for orally disintegrating compositions of emoxypine or a pharmaceutically acceptable salt, which overcomes above described problems and which further provide the advantageous property of allowing the active medicament to disintegrate or dissolve in the oral cavity without remaining substantial amounts of the active ingredient.

### Description of the invention

The main embodiment of the present invention is to provide a novel orally disintegrating composition comprising emoxypine or a pharmaceutically acceptable salt thereof and one or more pharmaceutically acceptable excipient which overcomes the above described problems in prior art and have additive advantages over them.

It has unexpectedly been found that the specific combination of the emoxypine with the combination of the super disintegrants provides an orally disintegrating composition which avoids the afore-mentioned disadvantages of the orally disintegrating compositions of the prior art.

According to the invention an emoxypine orally disintegrating composition is provided which is fundamentally comparable with the existing regular conventional solid dosage forms such as tablets or capsules, however with the unexpected benefits found with oral disintegration.

In one embodiment the orally disintegrating composition of emoxypine disintegrates in oral cavity in less than 90 seconds, preferably in less than 60 seconds, more preferably in less than 30 seconds.

In another embodiment of this invention, the orally disintegrating composition comprises emoxypine or a pharmaceutically acceptable salt in an amount of about 1% to 80% by weight of total formulation, preferably about 10% to 60%, the most preferably about 20% to 50%.

In a further embodiment, the present invention relates to the orally disintegrating composition comprising emoxypine or a pharmaceutically acceptable salt thereof, in an amount of 1 mg to 750mg. Preferably 50 mg to 500 mg, the most preferably 100 mg to 300 mg. Emoxypine is preferably in the form of succinate or HCl salt.

The orally disintegrating compositions of this invention further comprising at least one pharmaceutically acceptable excipient selected from the group comprising super disintegrants, diluents, binders, lubricants, glidants, sweeteners, flavouring agents, preservatives and coloring agents.

Suitable super disintegrants may include but not limited to croscarmellose sodium, crospovidone, low-substituted hydroxypropylcellulose, sodium starch glycollate and the like and mixtures thereof; preferably crosscarmellose sodium and/or crospovidone.

It has unexpectedly found that in this orally disintegrating composition having a weight ratio of emoxypine to super disintegrants, particularly crosscarmellose sodium, in the range of between 1:10 and 10:1 (w/w), has a synergistic effect over the disintegration time. Preferably the range is between 1:5 and 5:1 (w/w).

In one embodiment, the amount of crosscarmellose sodium is present about 0.1 to 30% by weight of total composition; preferably it is about 2 to 10% by weight of total composition.

In another embodiment, the amount of crospovidone is about 0.1 to 20 % by weight of total composition; preferably it is about 2 to 10 % by weight of total composition.

Suitable diluents may include but not limited to lactose, microcrystalline cellulose, mannitol, spray-dried mannitol, starch, sodium carbonate, sodium bicarbonate, calcium carbonate and the like and mixtures thereof; preferably spray-dried mannitol and/or microcrystalline cellulose.

It is reported that, spray-dried mannitol, has physical chemical properties that make it ideal for constituting the appropriate diluent for this invention. Because it dissolves easily and quickly in a little amount of water or saliva and its disintegrating rate is much faster than powder mannitol and other related saccharine excipients. It is highly compressible and it has optimum fluidity for direct compression processes. It has good dilution capacity due to the size and form of the particle, which makes it possible to accept large amounts of active ingredients that are not easily compressed. It is chemically very stable and non-hygroscopic.

In one embodiment, the amount of spray-dried mannitol is present about 1 to 60% by weight of total composition, preferably in an amount of about 10 to 40% by weight of total composition.

In one embodiment, when the ratio of microcrystalline cellulose is from 1 to 30% by weight of the total weight of the orally disintegrating composition, preferably from 10 to 15% by weight, said amount makes it possible to significantly improve compressibility, reduce friability and achieve a substantial reduction in disintegration time. Higher quantities have negative impact on the palatability of the formula and lower quantities worsen the disintegration time.

Surprisingly we have found that when the weight ratio of spray-dried mannitol to microcrystalline cellulose is in the range of between 1:10 and 10:1 (w/w), it has synergistic effect over the compressibility of emoxypine and disintegration time and stability of the orally disintegrating composition. Preferably the range is between 1:5 and 5:1 (w/w).

Suitable binders may include but not limited to polyvinylpyrrolidone, polyethylene glycol, polyvinyl alcohol, cellulose derivatives such as hydroxypropyl methyl cellulose, carboxy methyl cellulose, methyl cellulose, gelatin, polyvinylalcohol, carrageenan, guar gum, xanthan gum and the like and mixtures thereof; preferably polyvinylpyrrolidone and/or hydroxypropyl methyl cellulose. Binder content is about from 1 to 20%, preferably about from 2 to 10% by weight of total composition.

Suitable lubricants may include but not limited to magnesium strearate, calcium stearate, sodium stearyl fumarate, sodium lauryl sulphate, stearic acid and the like and mixtures thereof, preferably magnesium strearate and sodium lauryl sulfate. Lubricant content is about from 0,1 to 5% by weight of total composition.

Although the preferred lubricant is magnesium stearate, other less hydrophobic lubricants may be used to counter the hydrophobicity in certain cases such as a combination of magnesium stearate with sodium lauryl sulfate. The preffered ratio is about 10:1 to 7:1 (w/w).

Suitable glidants may include but not limited to colloidal silicon dioxide, talc, aluminium silicate and the like and mixtures thereof, preferably colloidal silicon dioxide. Glidant content is about from 0,1 to 5% by weight of total composition.

The orally disintegrating compositions of this invention may also include sweeteners and flavouring agents to improve patient compliance.

Suitable sweeteners may include but not limited to aspartame, sucralose, saccharin, sugars such as glucose, lactose, fructose and sugar alcohols such as mannitol, sorbitol, xylitol, erythritol and the like and mixtures thereof; preferably aspartame, sucralose and/or saccharin. Sweetener content is about from 0,01 to 5%, preferably about from 0.5 to 2% by weight of total composition.

Suitable flavoring agents may include but not limited to menthol, peppermint, cinnamon, chocolate, vanillin and fruit essences such as cherry, orange, strawberry, grape etc. and the like and mixtures thereof; preferably menthol and/or fruit essences. Flavouring agent content is about from 0.01 to 5%, preferably about from 0.5 to 2% by weight of total composition.

In a further embodiment, we have found that menthol, which due to its refreshing effect has a synergic effect with the spray-dried mannitol and a good tastemasking capacity due to its residual effect.

Suitable preservatives may include but not limited to methyl paraben and propyl paraben and their salts (such as sodium, potassium), sodium benzoate, citric acid, benzoic acid, butylated hydroxytoluene and butylated hydroxyanisole and the like and mixtures thereof; preferably citric acid. Preservative content is about from 0,01 to 5%, preferably about from 0.5 to 2% by weight of total composition.

Suiatable colouring agents may include but not limited to ferric oxide, FD&C (Food, Drug & Cosmetic) dyes, poncau and the like and mixtures thereof.

Optionally, a humidity absorbent agent may be added, such as precipitated silica in a proportion from 0.01 to 1% in weight of the total weight of the tablet, which may counteract the hydrophobicity of the active ingredient and improve the fluidity of the mixture.

In a further aspect, this present invention provides an orally disintegrating composition of emoxypine or a pharmaceutically acceptable salt thereof and one or more pharmaceutically acceptable excipients which is stable throughout the shelflife and which has high bioavailability.

The orally disintegrating compositions of this invention include tablets, sachets, minitablets, multilayer and multicoated tablets, pellets or powders which can be formulated in accordance with methods that are standard in the art. It is preferably in the form of tablets.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time.

The preferred shape of the orally disintegrating tablet composition of this invention may have a shape of a disk, circle, round, sphere, donut, bar, polygon, ellipse and the like. The preffered shape of the tablet is a flat round shape with bevelled edges.

The orally disintegrating compositions of the present invention may be prepared by conventional technology well known to those skilled in the art such as direct compression, dry granulation, wet granulation and the like. The orally disintegrating compositions of the present invention may also be prepared by other technologies such as spray drying, freeze drying, floss formation, molding, Zydis^{®} technology, Flashtab technology, OraSolv^{®} technology, DuraSolv^{®} technology, Wowtab^{™} (With Out Water quick-dissolve tablet) technology and the like. Preferably emoxypine or a pharmaceutically acceptable salt thereof, and other excipients are mixed together then the mixture is compressed to form tablets.

The manufacturing process is preferably performed by mixing the components, blending the mixture with glidant(s) and lubricant(s) and compressing the blended mixture to form tablets.

A preferred process for preparing the orally disintegrating compositions of the invention comprises the following steps:
(a) mixing emoxypine, or a pharmaceutically acceptable salt thereof with other excipients;
(b) blending the mixture with a glidant and lubricant;
(c) compressing the blended mixture to form tablets.

Another embodiment of the present invention is to provide an orally disintegrating pharmaceutical dosage form comprising emoxypine with one or more pharmaceutically acceptable excipient wich has a wide spectrum of pharmacological activities such as membranoprotection, neuroprotection, cardioprotection, antihypoxic, anxiolytic, nootropic, antiamnestic, antiatherogenic, antistress and antialcoholic actions.

Further, emoxypine is effective in patients with diabetes mellitus at early stages of diabetic foot syndrome when it is used in combination with alpha-lipoic acid. Emoxypine also improves learning and memory in hemorrhagic stroke and exerted influence on the locomotor activity.

It is also reported that emoxypine can be administered in combination with non-narcotic analgesics, in particular with Pentalgin, for relieving painful syndrome on the background of stress. Also it may be effective in combination with valproic acid (sodium valproate-Depakine^{®}) for treatment of patients with generalized epilepsy and as monotherapy in patients with first episode of febrile seizures.

Another action of emoxypine is eliminating anxiety and fear, recovering adequate reactions and the orientative-trying behavior, and lessens aggressiveness.

This invention is further defined by reference to the following example. Although the example is not intended to limit the scope of the present invention, it should be considered in the light of the description detailed above. It will be apparent to those skilled in the art that many modifications, both to materials and methods, may be practiced without departing from the scope of the invention.

To minimize the disintegration time and maximise the mechanical resistance of the tablets of this invention, this orally disintegrating tablet composition has been designed, made up of the following:

### Example

The orally disintegrating tablet composition comprises;
(a) about 1 to 80% by weight of emoxypine or pharmaceutically acceptable salt thereof,
(b) about 0,1 to 30% by weight of crosscarmellose sodium
(c) about 1 to 60% by weight of spray-dried mannitol
(d) about 1 to 30 % by weight of microcrystalline cellulose,
(e) about 1 to 20% by weight of polyvinylpyrollidone,
(f) about 0.01 to 5% by weight of colloidal silicon dioxide,
(g) about 0.1 to 5% by weight of magnesium stearate and sodium lauryl sulfate.
(h) about 0.01 to 5% by weight of saccharin sodium,
(i) about 0.01 to 5 % by weight of menthol and/or fruit essences.

The formulations of this example are manufactured according to the process described above in the description.

## Claims

1. An orally disintegrating composition comprising emoxypine or a pharmaceutically acceptable salt thereof and one or more pharmaceutically acceptable excipients.

2. The orally disintegrating composition according to claim 1, wherein the composition disintegrates in oral cavity in less than 90 seconds, preferably in less than 60 seconds.

3. The orally disintegrating composition according to claims 1 and 2, wherein emoxypine or a pharmaceutically acceptable salt thereof is present in an amount of about 1 to 80% by weight of total composition, preferably it is about 10 to 60% by weight of total composition, more preferably it is about 20 to 50% by weight of total composition.

4. The orally disintegrating composition according to claims 1 to 3, wherein the amount of emoxypine or a pharmaceutically acceptable salt is 1 mg to 750 mg, preferably 50 mg to 500 mg, more preferably 100 mg to 300mg.

5. The orally disintegrating composition according to claims 1 to 4, wherein emoxypine is in the form of a succinate salt or HCl salt.

6. The orally disintegrating composition according to claims 1 to 5, wherein the one or more pharmaceutically acceptable excipients are selected from the group comprising super disintegrants, diluents, binders, lubricants, glidants, sweeteners, flavouring agents, preservatives and coloring agents.

7. The orally disintegrating composition according to claim 6, wherein the super disintegrants are selected from the group comprising croscarmellose sodium, crospovidone, low-substituted hydroxypropylcellulose, sodium starch glycollate and the like and mixtures thereof.

8. The orally disintegrating composition according to claims 1 to 7, wherein the weight ratio of emoxypine to super disintegrants is in the range of between 1:10 and 10:1 (w/w).

9. The orally disintegrating composition according to claim 7 and 8, wherein the super disintegrant is preferably crosscarmellose sodium.

10. The orally disintegrating composition according to claim 9, wherein the amount of crosscarmellose sodium is present in an amount of about 0.5 to 30% by weight of total composition, preferably it is about 3 to 15% by weight of total composition.

11. The orally disintegrating composition according to claim 6, wherein the diluents are preferably spray-dried mannitol and/or microcrystalline cellulose.

12. The orally disintegrating composition according to claim 11, wherein the amount of spray-dried mannitol is in an amount of about 1 to 60% by weight of total composition, preferably it is about 10 to 40% by weight of total composition.

13. The orally disintegrating composition according to claim 11, wherein the amount of microcrystalline cellulose is in an amount of about 1 to 30% by weight of total composition, preferably it is about 10 to 15% by weight of total composition.

14. The orally disintegrating composition according to claims 11 to 13, wherein the weight ratio of spray-dried mannitol to microcrystalline cellulose is in the range of between 1:10 and 10:1 (w/w).

15. The orally disintegrating composition according to claim 6, wherein the binders are preferably polyvinylpyrollidone and/or hydroxypropyl methyl cellulose,

16. The orally disintegrating composition according to claim 6, wherein the lubricants are preferably magnesium strearate and sodium lauryl sulfate.

17. The orally disintegrating composition according to claim 16, wherein the weight ratio of magnesium stearate to sodium lauryl sulfate is between 10:1 and 7:1.

18. The orally disintegrating composition according to claim 6, wherein the glidants is preferably colloidal silicon dioxide.

19. The orally disintegrating composition according to claim 6, wherein the sweetners are preferably aspartame, sucralose and/or saccharin.

20. The orally disintegrating composition according to claim 6, wherein the flavouring agents are preferably menthol and/or fruit essences.

21. The orally disintegrating composition according to any preceding claim comprising;
(a) about 1 to 80% by weight of emoxypine or pharmaceutically acceptable salt thereof,
(b) about 0,1 to 30% by weight of crosscarmellose sodium
(c) about 1 to 60% by weight of spray-dried mannitol
(d) about 1 to 30 % by weight of microcrystalline cellulose,
(e) about 1 to 20% by weight of polyvinylpyrollidone,
(f) about 0.01 to 5% by weight of colloidal silicon dioxide,
(g) about 0.1 to 5% by weight of magnesium stearate and sodium lauryl sulfate.
(h) about 0.01 to 5% by weight of saccharin sodium,
(i) about 0.01 to 5 % by weight of menthol and/or fruit essences.

22. A process for preparing orally disintegrating compositions according to any preceding claim comprising;
(a) mixing emoxypine, or a pharmaceutically acceptable salt with other excipients;
(b) blending the mixture with a glidant and a lubricant;
(c) compressing the blended mixture to form tablets.
